# EUROPEAN PATENT APPLICATION

(11) **EP 3 488 816 A1**
(43) Date of publication of application: **29.05.2019**
(21) Application number: 18208632.2
(22) Date of filing: 27.11.2018
(51) Int. Cl.: A61F 2/04, A61F 2/86

(54) **COLORECTAL STENTS**

(30) Priority: 28.11.2017 US 201762591720 P; 24.09.2018 US 201816139516
(71) Applicant: Covidien LP, Mansfield, MA 02048 (US)
(72) Inventor: JADHAV, Amarsinh Deeliprao, 500050 Hyderabad (IN)
(74) Representative: Maschio, Antonio

(57) **Abstract**

A stent for use in colorectal surgery is expandable and includes an inner surface having a layer thereon to prevent fecal matter from being trapped in the cells/openings of the stent. In embodiments, the stent is placed within the colon at the site of an anastomosis. The layer on the inner surface of the stent also prevents leakage at the site of an anastomosis. In embodiments, the stent is self-expanding, and expands radially to engage an inner wall of a lumen, in embodiments the colon. In other embodiments, the stent is expanded by a balloon to engage the inner wall of a lumen, in embodiments the colon. Methods for using stents of the present disclosure are also provided.

## Description

### CROSS-REFERENCE TO RELATED APPLICATION

This application claims the benefit of and priority to U.S. Provisional Patent Application No. 62/591,720 filed November 28, 2017, the entire disclosure of which is incorporated by reference herein.

### TECHNICAL FIELD

The present disclosure relates generally to surgical apparatuses for use in gastrointestinal surgery, and more particularly, the present disclosure relates to stents that may be used with patients undergoing a colorectal procedure in conjunction with an end-to-end anastomosis.

### BACKGROUND

A stent is an elongated device used to support a luminal wall. Various types of stent architectures are within the purview of those skilled in the art, including many designs that encompass a filament or number of filaments, such as a wire or wires, wound or braided into a particular configuration. Included among these wire stent configurations are braided stents. Braided stents tend to be very flexible, having the ability to be placed in tortuous anatomy and still maintain patency. The flexibility of braided stents makes them particularly well-suited for use in intraluminal delivery where the lumen of the vessel becomes contorted and irregular both before and after placement of the stent.

In the field of gastrointestinal surgery, after resection of diseased tissue, stents may be used to assist with maintenance of gastrointestinal continuity upon re-anastomosis.

Stents to protect the anastomosis from bowel contents during the healing phase, capable of reducing the risk of anastomotic leaks and preventing tissue growth at the site of the anastomosis, thereby reducing future blockage, remain desirable.

### SUMMARY

The present disclosure relates to a stent for use in the intestines that may be deployed as part of a colorectal procedure including an end-to-end anastomosis of the intestine. Methods for using such a stent are also provided.

A stent of the present disclosure includes, in embodiments, a tubular member having a proximal portion and a distal portion and defining a longitudinal axis and a lumen extending between the proximal portion and the distal portion of the tubular member. The tubular member includes an outer surface and an inner surface and a layer along the inner surface of the tubular member, the layer having ends which extend from the lumen of the tubular member, the ends of the layer being affixed to the outer surface of the tubular member thereby forming cuffs at the proximal portion and the distal portion of the tubular member. The stent and the layer are both formed of a material which permits expansion of the stent in a patient's body.

In some embodiments, the stent is configured to be expanded by a balloon.

In other embodiments, the stent is self-expanding. For example, the stent may be formed of a shape memory material.

In embodiments, the layer is formed of an elastomeric polymer. Suitable elastomeric polymers include, for example, a natural or synthetic rubber, polyurethane, polyisoprene, polybutadiene, chloroprene, polyisobutylene, combinations thereof, or copolymers thereof.

Methods of the present disclosure include, in embodiments, introducing an impermeable stent defining a stent lumen into a lumen of a body vessel in a compressed configuration and positioning the impermeable stent at a site of an anastomosis such that the stent extends across the site of the anastomosis. The impermeable stent is then radially expanded to an enlarged circumference, thereby placing the outer surface of the impermeable stent in contact with an inner wall of the body vessel across the site of the anastomosis, thereby anchoring the impermeable stent within the body vessel.

In embodiments, introducing the impermeable stent includes advancing the impermeable stent along a guidewire.

In some embodiments, radially expanding the impermeable stent includes expanding the impermeable stent with a balloon.

In other embodiments, the impermeable stent is formed from a self-expanding material and radially expanding the impermeable stent includes retracting a retractable sheath from about the impermeable stent to permit the impermeable stent to self-expand.

In some embodiments, the impermeable stent is introduced to the site of the anastomosis at the time of forming the anastomosis. In other embodiments, the impermeable stent is introduced to the site of the anastomosis after formation of the anastomosis.

### DESCRIPTION OF THE DRAWINGS

Various embodiments of the presently disclosed colorectal stents are described herein with reference to the drawings wherein:
FIG. 1A is a side, perspective view of an exemplary embodiment of a stent in accordance with the present disclosure;
FIG. 1B is a cross-sectional view of the stent of FIG. 1A;
FIG. 1C is a side, perspective view of an alternate exemplary embodiment a stent in accordance with the present disclosure;
FIG. 2 is a perspective view of an anastomosis of a patient's colon;
FIG. 3 is a perspective, partial cross-sectional view of the anastomosis site taken along sectional line 3-3 of FIG. 2;
FIG. 4 is a side view showing the placement of the stent of FIG. 1A within the patient's colon at the site of the anastomosis;
FIG. 5A is a side view showing the self-expansion of the stent of FIG. 1A within the patient's colon at the site of the anastomosis;
FIG. 5B is a side view showing expansion of the stent of FIG. 1A with a balloon within the patient's colon at the site of the anastomosis; and
FIG. 6 is a perspective view of the stent of FIG. 1C shown in phantom within the patient's colon at the site of the anastomosis.

### DETAILED DESCRIPTION

The present disclosure provides a stent for use in colorectal surgical procedures. As used herein with reference to the present disclosure, colorectal surgical procedures encompass surgeries of the colon, rectum and anus, and refer to procedures including, in embodiments, end-to-end anastomoses of the colon to remove diseased sections thereof.

As used herein, the term distal refers to that portion of a surgical apparatus, including a stent of the present disclosure, which is farthest from the user, while the term proximal refers to that portion of the surgical apparatus of the present disclosure which is closest to the user.

The stent of the present disclosure includes a hollow tubular member having a wall defining a lumen along its longitudinal axis. The stent of the present disclosure is expandable, i.e., it may be introduced into the intestinal lumen in a reduced circumference, sometimes referred to herein, in embodiments, as a compressed configuration, and thereafter radially expands to an enlarged circumference, which places the outer surface of the stent in contact with the inner wall of the intestine at the site of placement, thereby anchoring the stent in place at the desired location within the intestine.

A stent of the present disclosure may be a balloon expandable stent or a self-expanding stent. Stents of the present disclosure may be formed of any biocompatible material, including polymeric materials and metallic materials such as stainless steel, titanium, shape memory alloys, combinations thereof, and the like. The stents can be any size, shape, internal structure, etc., and include braided stents or other forms of stents such as a laser-cut stents, knitted stents, and the like, as long as the stent is expandable within the lumen of the intestine.

In embodiments, the stent is a self-expanding, open-celled, tubular stent formed of a shape memory material. Such stents may be braided from filaments formed of suitable self-expanding, shape memory or superelastic material. In embodiments, suitable shape memory materials include shape memory metals and metal alloys such as Nitinol, or the like.

In embodiments, the braid formed of such shape memory materials is heat-treated or "heat-set" at a high temperature in order to reduce internal stresses in the filaments and/or increase or impart a self-expanding capability of the stent. Filaments making up the tubular body of a stent that has been heat set are in their least-stressed or a reduced-stressed state when the stent is in the configuration it was in during heat setting. Such a least-stressed or reduced-stressed state can include an expanded or fully expanded state, thereby making the stent self-expanding.

Embodiments of the present disclosure will now be described in detail with reference to the drawings, in which like reference numerals designate identical or corresponding elements in each of the several views. In the following description, well-known functions or constructions are not described in detail to avoid obscuring the present disclosure in unnecessary detail.

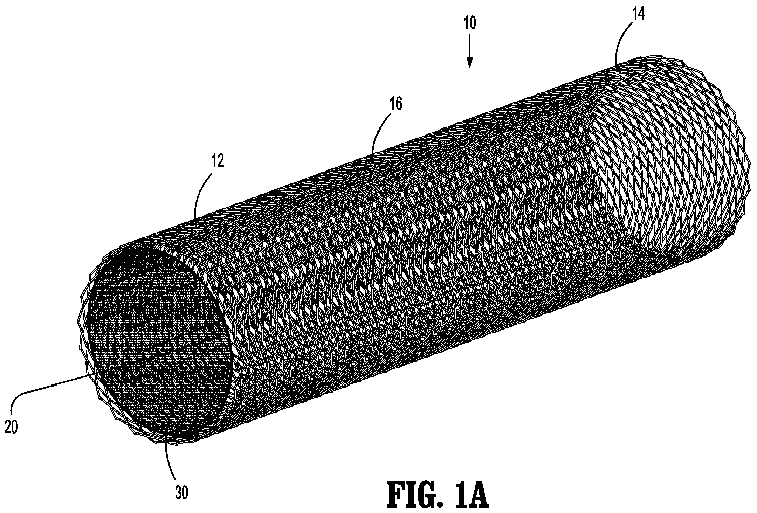

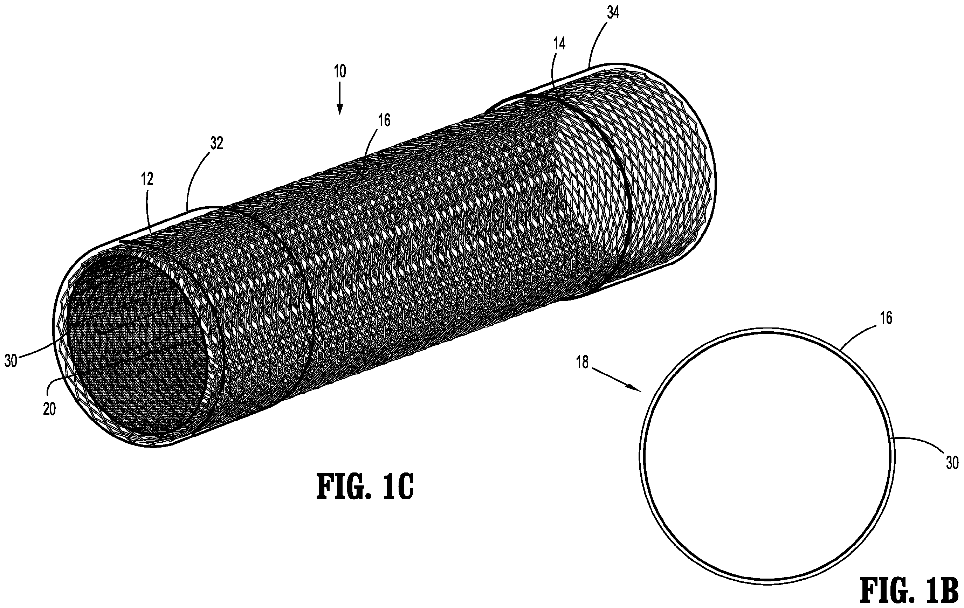

FIGs. 1A and 1C illustrate perspective views of a stent 10 in accordance with embodiments of the present disclosure. FIG. 1B is a cross-sectional view of the stent 10 of FIG. 1A. The stent 10 has ends 12, 14, an outer surface 16, and an inner surface 18. The inner surface 18 of the stent 10 forms a lumen 20 therethrough connecting the ends 12, 14 of the stent 10. The inner surface 18 of the stent 10 has a layer 30 thereon to prevent fecal matter from being trapped in the cells/openings of the stent 10. As shown in FIG. 1C, in some embodiments, the layer 30 protrudes from the ends 12, 14 of the lumen 20 of the stent 10, with the portions of the layer 30 protruding from the ends 12, 14 affixed to the outer surface 16 of the stent 10 to form cuffs 32, 34 at the ends 12, 14 of the stent 10.

The layer 30 may be made from any elastomeric polymer capable of expanding with the stent 10. Non-limiting examples of suitable elastomeric polymers include natural or synthetic rubbers, polyurethane, polyisoprene, polybutadiene, chloroprene, polyisobutylene, as well as combinations and copolymers thereof. The layer 30 may be impermeable to, among other things, alimentary tract contents such as, for example, bile, water, and/or fecal matter. This prevents the openings/cells of the stent 10 from becoming clogged with fecal matter, as well as reduces leakage at the site of the anastomosis, thereby imparting impermeability to the stent 10.

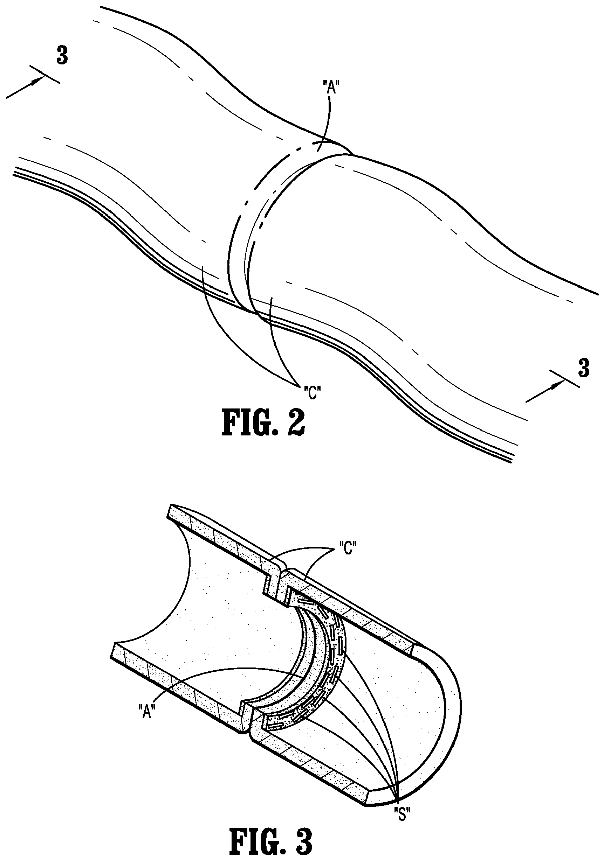

As noted above, in embodiments, the stents of the present disclosure may be placed at the site of an end-to-end anastomosis. Such an anastomosis "A" of the colon "C" is depicted in FIGs. 2 and 3. FIG. 3 shows the interior of the colon along axis 3-3 in FIG. 2 after the anastomosis "A" has been performed, with staples "S" attaching the two ends of the colon "C" in the end-to-end anastomosis "A".

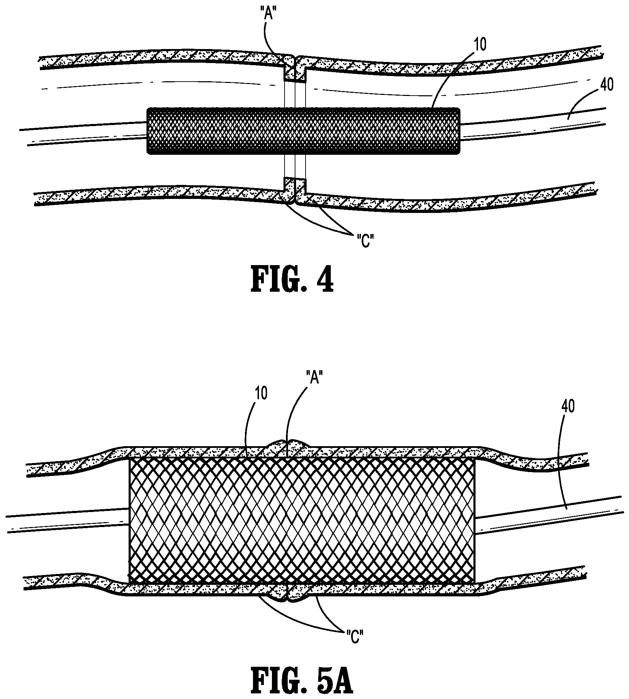

Once the anastomosis "A" has been performed, the stent 10 may be introduced within the colon "C" to the site of the anastomosis "A" by any suitable delivery device within the purview of those skilled in the art, such as guidewires or catheters, including balloon catheters. In embodiments, as depicted in FIG. 4, a guidewire 40 may be introduced into the colon "C" and the stent 10 may travel to the site of the anastomosis "A" along the guidewire 40 in a compressed configuration. In embodiments, the stent 10 may be covered by a retractable sheath (not shown) for introduction within the colon "C" to the site of the anastomosis "A".

Once in the proper location, as depicted in FIG. 5A, a self-expanding stent 10 of the present disclosure may radially expand to its enlarged circumference into engagement with the inner wall of the colon "C" thereby anchoring itself within the colon "C" at the site of the anastomosis "A". Where the stent 10 is covered by a retractable sheath (not shown), retraction of the retractable sheath permits this self-expansion.

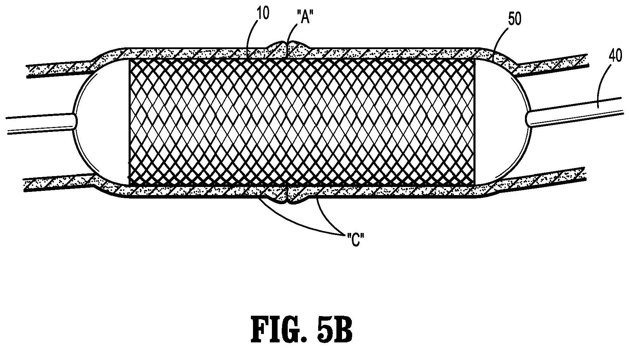

In other embodiments, as depicted in FIG. 5B, a balloon expandable stent 10 of the present disclosure may be expanded by a balloon 50 within the colon "C" at the site of the anastomosis "A".

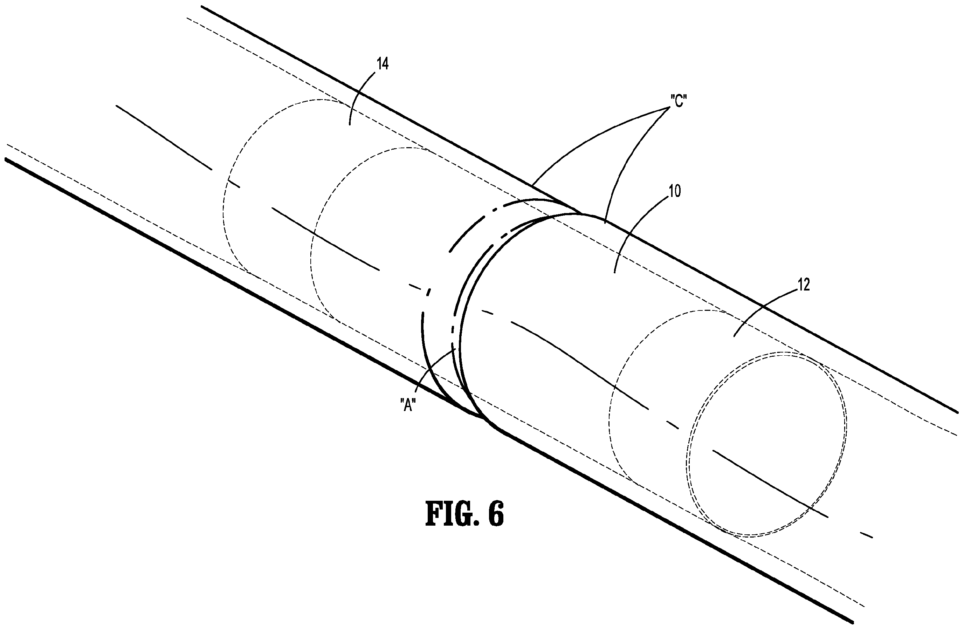

As shown in FIG. 6, after deployment of the stent 10 at the site of the anastomosis "A", the stent 10 (shown in phantom) will maintain patency of the colon "C" at the site of the anastomosis "A". The stent 10 will also minimize contact of fecal matter with the site of the anastomosis "A" to minimize the possibility of leakage at the site of the anastomosis "A".

In addition to the procedure described above where the stent 10 is deployed within the colon "C" at the time of performance of the anastomosis "A", it is also contemplated that the stent 10 may be deployed after performance of an anastomosis, for example, in a patient where tissue growth has started at the anastomosis site and the patient is beginning to experience difficulty in passing fecal matter.

While the stent device in the exemplary embodiments presented herein may be used within the colon, rectum and anus, it is understood that these are exemplary embodiments presented to demonstrate aspects of the present disclosure. The description provided herein may refer to the deployment of a stent device in particular to the colon and rectum, but it is also understood that aspects of the present disclosure may be employed within other parts of the gastrointestinal tract, such as the small intestine, biliary tract, esophagus, and/or stomach.

It will be understood that various modifications may be made to the embodiments disclosed herein. For example, other methods for introducing stents of the present disclosure into the body of a patient may be used. Additionally, other stent shapes may be used. Further, the terminology of similar components with the various embodiments should not be construed as specific to any particular embodiment. Thus, the above description should not be construed as limiting, but merely as exemplifications of preferred embodiments. Those skilled in the art will envision other modifications within the scope and spirit of the claims appended hereto.

The invention may be described by reference to the following numbered paragraphs:-
1. A stent comprising:
   a tubular member having a proximal portion and a distal portion and defining a longitudinal axis and a lumen extending between the proximal portion and the distal portion of the tubular member, the tubular member including an outer surface and an inner surface; and
   a layer along the inner surface of the tubular member, the layer having ends which extend from the lumen of the tubular member, the ends of the layer being affixed to the outer surface of the tubular member thereby forming cuffs at the proximal portion and the distal portion of the tubular member,
   the stent and the layer both being formed of a material which permits expansion of the stent in a patient's body.
2. The stent of paragraph 1, wherein the stent is configured to be expanded by a balloon.
3. The stent of paragraph 1, wherein the stent is self-expanding.
4. The stent of paragraph 3, wherein the stent is formed of a shape memory material.
5. The stent of paragraph 1, wherein the layer is formed of an elastomeric polymer.
6. The stent of paragraph 5, wherein the elastomeric polymer is a natural or synthetic rubber, polyurethane, polyisoprene, polybutadiene, chloroprene, polyisobutylene, combinations thereof, or copolymers thereof.
7. A method comprising:
   introducing an impermeable stent defining a stent lumen into a lumen of a body vessel in a compressed configuration;
   positioning the impermeable stent at a site of an anastomosis such that the stent extends across the site of the anastomosis; and
   radially expanding the impermeable stent to an enlarged circumference, thereby placing the outer surface of the impermeable stent in contact with an inner wall of the body vessel across the site of the anastomosis, thereby anchoring the impermeable stent within the body vessel.
8. The method of paragraph 7, wherein the impermeable stent includes a tubular member having a proximal portion and a distal portion and defining a longitudinal axis and a lumen between the proximal portion and the distal portion of the tubular member, the tubular member including an outer surface and an inner surface and a layer along the inner surface of the tubular member.
9. The method of paragraph 8, wherein the layer along the inner surface of the tubular member has ends which extend from the lumen of the tubular member, the ends of the layer being affixed to the outer surface of the tubular member thereby forming cuffs at the proximal portion and the distal portion of the tubular member.
10. The method of paragraph 8, wherein the layer along the inner surface of the tubular member is formed of an elastomeric polymer.
11. The method of paragraph 10, wherein the elastomeric polymer is a natural or synthetic rubber, polyurethane, polyisoprene, polybutadiene, chloroprene, polyisobutylene, combinations thereof, or copolymers thereof.
12. The method of paragraph 7, wherein introducing the impermeable stent includes advancing the impermeable stent along a guidewire.
13. The method of paragraph 7, wherein radially expanding the impermeable stent includes expanding the impermeable stent with a balloon.
14. The method of paragraph 7, wherein the impermeable stent is formed from a self-expanding material.
15. The method of paragraph 14, wherein the impermeable stent is formed of a shape memory material.
16. The method of paragraph 14, wherein radially expanding the impermeable stent includes retracting a retractable sheath from about the impermeable stent to permit the impermeable stent to self-expand.
17. The method of paragraph 7, wherein the impermeable stent is introduced to the site of the anastomosis at the time of forming the anastomosis.
18. The method of paragraph 7, wherein the impermeable stent is introduced to the site of the anastomosis after formation of the anastomosis.

## Claims

1. A stent comprising:
a tubular member having a proximal portion and a distal portion and defining a longitudinal axis and a lumen extending between the proximal portion and the distal portion of the tubular member, the tubular member including an outer surface and an inner surface; and
a layer along the inner surface of the tubular member, the layer having ends which extend from the lumen of the tubular member, the ends of the layer being affixed to the outer surface of the tubular member thereby forming cuffs at the proximal portion and the distal portion of the tubular member,
the stent and the layer both being formed of a material which permits expansion of the stent in a patient's body.

2. The stent of claim 1, wherein the stent is configured to be expanded by a balloon.

3. The stent of claim 1, wherein the stent is self-expanding.

4. The stent of claim 3, wherein the stent is formed of a shape memory material.

5. The stent of any preceding claim, wherein the layer is formed of an elastomeric polymer.

6. The stent of claim 5, wherein the elastomeric polymer is a natural or synthetic rubber, polyurethane, polyisoprene, polybutadiene, chloroprene, polyisobutylene, combinations thereof, or copolymers thereof.
